# EUROPÄISCHE PATENTSCHRIFT

(11) **EP 1 141 277 B1**
(45) Veröffentlichungstag und Bekanntmachung des Hinweises auf die Patenterteilung: **07.05.2003**
(21) Anmeldenummer: 99967983.0
(22) Anmeldetag: 23.12.1999
(51) Int. Cl.: C12N 15/11, A61K 31/7125, C07H 21/00, A61P 35/02

(54) **ANTISENSE-OLIGONUKLEOTID ZUR HEMMUNG DER EXPRESSION DES ADHÄSIONSMOLEKÜLS VERY LATE ANTIGEN 4 (VLA-4) IN MENSCHLICHEN ZELLEN**
ANTISENSE OLIGONUCLEOTIDE FOR INHIBITING THE EXPRESSION OF THE ADHESION MOLECULE VERY LATE ANTIGEN 4 (VLA-4) IN HUMAN CELLS
OLIGONUCLEOTIDE ANTISENS POUR L'INHIBITION DE L'EXPRESSION DE LA MOLECULE D'ADHESION VLA-4 (VERY LATE ANTIGEN 4) DANS LES CELLULES HUMAINES

(30) Priorität: 29.12.1998 DE 19860642
(43) Veröffentlichungstag der Anmeldung: 10.10.2001
(73) Patentinhaber: Deutsches Krebsforschungszentrum Stiftung des öffentlichen Rechts, 69120 Heidelberg (DE)
(72) Erfinder: HAAS, Rainer, D-40237 Düsseldorf (DE); KRONENWETT, Ralf, D-40237 Düsseldorf (DE); LICHTERFELD, Mathias, D-69115 Heidelberg (DE)
(74) Vertreter: Müller-Boré & Partner Patentanwälte
(86) Internationale Anmeldenummer: EP9910387
(87) Internationale Veröffentlichungsnummer: WO00039292

(56) Entgegenhaltungen:
- US-A- 5 968 826
- LICHTERFELD M. ET AL: "Functional analyses of antisense oligonucleotide-mediated downregulation of the integrin VBLA-4 in normal and leukemic hematopoietic cells." ANNALS OF HEMATOLOGY , Bd. 77, Nr. SUPPL. 2, 1998, Seite S152 XP000907520 ISSN: 0939-5555 & ANNUAL CONGRESS OF THE GERMAN AND AUSTRIAN SOCIETIES OF HEMATOLOGY AND ONCOLOGY;FRANKFURT, GERMANY; OCTOBER 25-28, 1998 ,
- FOSTER C A : "VCAM-1/alpha 4-integrin adhesion pathway: Therapeutic target for allergic inflammatory disorders" JOURNAL OF ALLERGY AND CLINICAL IMMUNOLOGY, (DEC 1996) VOL. 98, NO. 6, PART 2, SUPP. [S], PP. S270-S277., XP000907565
- KRONENWETT R ET AL: "Functional analyses of antisense oligonucleotide-mediated downregulation of the integrin VLA-4 in normal and leukemic hematopoietic cells." 40TH ANNUAL MEETING OF THE AMERICAN SOCIETY OF HEMATOLOGY;MIAMI BEACH, FLORIDA, USA; DECEMBER 4-8, 1998 BLOOD NOV. 15, 1998, Bd. 92, Nr. 10 SUPPL. 1 PART 1-2, 15. November 1998 (1998-11-15), Seite 586A XP002139341 ISSN: 0006-4971
- CRADDOCK, C. ET AL.: "Antibodies to VLA4integrin mobilize long-term repopulating cells and augment cytokine-induced mobilization in primates and mice" BLOOD, Bd. 90, Nr. 12, 15. Dezember 1997 (1997-12-15), Seiten 4779-4788, XP002139342 ISSN: 0006-4971 in der Anmeldung erwähnt
- PAPAYANNOPOULOU, T. & NAKAMOTO, B.: "Peripheralization of hemopoietic progenitors in primates treated with anti-VLA4 integrin" PROCEEDINGS OF THE NATIONAL ACADEMY OF SCIENCES OF USA., Bd. 90, Oktober 1993 (1993-10), Seiten 9374-9378, XP002139343 ISSN: 0027-8424 in der Anmeldung erwähnt

## Beschreibung

Die vorliegende Erfindung betrifft eine Nukleinsäure, enthaltend eine Nukleotidsequenz, welche durch Bindung an ein Transkriptionsprodukt des für die α4-Kette des Adhäsionsmoleküls VLA-4 kodierenden Gens die Expression des Adhäsionsmoleküls VLA-4 in Säugerzellen vollständig oder mindestens teilweise inhibiert, einen Vektor, der die Nukleinsäure enthält, sowie eine pharmazeutische Zusammensetzung, welche die Nukleinsäure oder den Vektor enthält und beispielsweise zur Mobilisierung von Blutstammzellen in Säugern, zur Behandlung von malignen hämatologischen Erkrankungen sowie zur Behandlung von pathologischen Entzündungsreaktionen in Säugern eingesetzt werden kann.

Das Adhäsionsmolekül "very late antigen 4" (VLA-4) ist ein Protein der Integrin-Familie, das beispielsweise auf mononukleären Leukozyten (Lymphozyten, Monozyten), auf eosinophilen und basophilen Granulozyten sowie auf CD34⁺ hämatopoetischen Stammzellen exprimiert wird. Die Interaktion von VLA-4 mit seinen Liganden VCAM-1 auf Endothelzellen und Fibronektin der extrazellulären Matrix spielt eine zentrale Rolle bei der Einwanderung von Leukozyten in entzündetes Gewebe. Beispiele für pathologische Entzündungsreaktionen sind u.a. chronisch-entzündliche Darmerkrankungen, rheumatoide Athritis, Organabstoßungsreaktionen und allergische Reaktionen. Außerdem spielt VLA-4 eine wichtige Rolle beim sog. "Homing" (von engl. to home; das Ziel ansteuern, nach Hause finden; d.h. die durch zell- bzw. gewebsspezifische Rezeptoren vermittelte Einnistung von Leukozyten oder Stammzellen in das Zielgewebe oder -organ) und bei der Proliferation und Reifung von CD34⁺-Zellen im Knochenmark. Eine Blockierung der Adhäsion durch VLA-4-gerichtete monoklonale Antikörper (mAK) führt zur Mobilisierung von hämatopoetischen Vorläuferzellen in das periphere Blut (Papayannopoulou et al., 1993). Die Gewinnung von CD34⁺-Zellen aus dem peripheren Blut spielt klinisch eine wichtige Rolle, da eine myeloablative Hochdosistherapie mit Transplantation autologer oder allogener Blutstammzellen für einzelne Leukämieformen, maligne Lymphome sowie solide Tumore mit ungünstigen Prognosefaktoren zu einer kurativen Therapie geworden ist.

CD34⁺ hämatopoetische Stammzellen werden derzeit durch eine cytotoxische Chemotherapie mit nachfolgender Gabe rekombinanter hämatopoetischer Wachstumsfaktoren in das periphere Blut mobilisiert (Haas et al., 1995). Ein experimenteller Mobilisierungsansatz ist die systemische Gabe von VLA-4-gerichteten monoklonalen Antikörpern. Bei Primaten konnten dadurch Blutstammzellen in das periphere Blut mobilisiert werden (Papayannopoulou et al., 1993). Die Kombination von anti-VLA-4 mAK mit rekombinanten Wachstumsfaktoren führte zu einem synergistischen Effekt bei der Mobilisierung hämatopoetischer Stammzellen in Primaten (Craddock et al., 1997).

Zur Behandlung chronisch-entzündlicher und allergischer Erkrankungen werden derzeit hauptsächlich Corticosteroide eingesetzt. Als molekulare Therapieansätze sind Antisense-Oligonukleotide gegen ein anderes Adhäsionsmolekül, dem ICAM-1, bereits in einer klinischen Phase IIa Studie bei Patienten mit Morbus Crohn (Yacyshyn et al., 1998) verwendet worden. VLA-4-Antagonisten bei pathologischen Entzündungsreaktionen befinden sich gegenwärtig im experimentellen Stadium und in ersten klinischen Studien. Zum Beispiel wurden VLA-4-gerichtete monoklonale Antikörper bei Mäusen und Primaten zur Inhibierung entzündlicher und allergischer Reaktionen eingesetzt (Podolsky et al., 1993; Young et al., 1993; Chisholm et al., 1993; Yednok et al., 1992). Von Fibronektinabgeleitete Peptide, die spezifisch an VLA-4 binden können, zeigten in Mäusen anti-inflamatorische Eigenschaften (Ferguson et al., 1991; Henes et al., 1994; Wahl et al., 1994). Solche Peptide werden jetzt auch an ersten klinischen Studien bei Patienten mit Asthma eingesetzt.

Durch die bisherige etablierte Methode der Blutstammzellmobilisierung mit Chemotherapie und rekombinanten Wachstumsfaktoren können bei etwa 15% der Patienten aufgrund früherer cytotoxischer Therapien keine CD34⁺-Zellen mobilisiert werden. Daher muß nach alternativen Ansätzen gesucht werden. Zur Mobilisierung von CD34⁺-Zellen bei gesunden allogenen Spendern werden derzeit rekombinante Wachstumsfaktoren alleine ohne Chemotherapie verwendet. Diese Wachstumsfaktoren zeigen Nebenwirkungen, wogegen Oligonukleotide in bisherigen Studien sehr gut verträglich waren. Durch die Kombination von rekombinanten Wachstumsfaktoren mit VLA-4-Antisense-Oligonukleotiden können möglicherweise größere CD34⁺-Zellausbeuten erzielt werden, was zu einem schnelleren Anwachsen des Stammzelltransplantats führt.

Zur Behandlung chronisch-entzündlicher und allergischer Erkrankungen werden derzeit Corticosteroide verwendet, die eine Vielzahl von starken Nebenwirkungen besitzten. Daher sind nebenwirkungsarme und kausale Therapieansätze nötig. Neuartige molekulare Therapieansätze sind jedoch zu wenig untersucht, um Aussagen über spezifische Nachteile treffen zu können. Grundsätzliche Nachteile von Antikörpern sind die schwierige Herstellung unter entsprechenden Qualitätsstandards. Peptide und Proteine können immunogen wirken. Beides sind Nachteile, die bei der Verwendung von Nukleinsäuren als therapeutische Moleküle nicht vorhanden sind.

In der Literatur wurde allgemein offenbart, es gäbe Antisense-Oligonukleotide die es erlauben die VLA-4 (α4-Kette) Expression in hämatopoietischen Zellen um 30% sowie dieselbe auch in leukämischen myeloiden sowie lymphoiden Zellinien zu verringern. Allerdings sind in diesem Dokument keine spezifische Nukleotidsequenzen des Antisense-Moleküls selbst beschrieben worden (LICHTERFELD M. ET AL: 'Functional analyses of antisense oligonucleotidemediated downregulation of the integrin VBLA-4 in normal and leukemic hematopoietic cells.' ANNALS OF HEMATOLOGY, Bd. 77, Nr. SUPPL. 2, 1998, Seite S152).

Somit liegt der vorliegenden Erfindung die Aufgabe zugrunde, ein neues System zur Inhibierung bzw. Herunterregulierung der Expression des Adhäsionsmoleküls VLA-4 in Säugerzellen bereitzustellen.

Diese Aufgabe wird durch die in den Ansprüchen gekennzeichneten Ausführungsformen der vorliegenden Erfindung gelöst.

Insbesondere wird eine Nukleinsäure, enthaltend eine Nukleotidsequenz, welche durch Bindung an ein Transkriptionsprodukt des für die α4-Kette des Adhäsionsmoleküls VLA-4 kodierenden Gens die Expression des Adhäsionsmoleküls VLA-4 in humanen Zellen, die aus der Gruppe, bestehend aus Monozyten, Granulozyten, Lymphomzellen, Zellen des Rhabdomyosarkoms und des malignen Melanoms ausgewählt sind, vollständig oder mindestens um 30% inhibiert, bereitgestellt.

Der Begriff "Adhäsionsmolekül" bedeutet, daß das Molekül eine Wechselwirkung bzw. Bindung zwischen zwei Zellen vermittelt. Das Adhäsionsmolekül VLA-4 ist ein Protein, das zur Familie der Integrine zählt, und wird beispielsweise auf mononukleären Leukozyten wie Lymphozyten und Monozyten, auf eosinophilen und basophilen Granulozyten, auf CD34⁺ hämatopoetischen Stammzellen sowie teilweise auf leukämischen Zellen bei akuten und chronischen Leukämien exprimiert. Außerem wird VLA-4 auf bestimmten nicht-hämatopoetischen Tumorzellen (z.B. Rhabdomyosarkom, Melanom) exprimiert.

Der Begriff "Transkriptionsprodukt" umfaßt alle diejenigen Nukleinsäuren, die durch Transkription des entsprechenden Gens mittels der Transkriptionssysteme der Zelle primär hergestellt, oder mittels der weiteren Enzymsysteme zur Bildung der 5'-Endkappe und des 3'-Poly-A⁺-Schwanzes sowie des Spleißapparates zur reifen mRNA prozessiert werden.

Der Begriff "Bindung" bedeuted, daß die erfindungsgemäße Nukleinsäure und das Transkriptionsprodukt des für die α4-Kette des Adhäsionsmoleküls VLA-4 kodierenden Gens in der Lage sind, in spezifischer Weise miteinander eine Wechselwirkung einzugehen, was die Bildung kovalenter chemischer Bindungen, elektrostatischer Wechselwirkungen aufgrund von Salzbrücken, Wasserstoffbrückenbindungen und/oder hydrophober Wechselwirkungen sowie eine Bindung aufgrund von Basenstapelungen umfassen kann. Insbesondere sind Wasserstoffbrückenbindungen und hydrophobe Wechselwirkungen die Triebkräfte für eine Bindung durch Hybridisierung zwischen der erfindungsgemäßen Nukleinsäure und dem Transkriptionsprodukt des für die α4-Kette des Adhäsionsmoleküls VLA-4 kodierenden Gens. Eine Hybridisierung zwischen der erfindungsgemäßen Nukleinsäure und dem Transkriptionsprodukt des für die α4-Kette des Adhäsionsmoleküls VLA-4 kodierenden Gens kann beispielsweise dann erfolgen, wenn mindestens ein Teil der Nukleotidsequenz der erfindungsgemäßen Nukleinsäure mindestens teilweise zu mindestens einem Teil der für die α4-Kette des Adhäsionsmoleküls VLA-4 kodierenden mRNA komplementär ist.

In einer bevorzugten Ausführungsform der vorliegenden Erfindung sind die humanen Zellen ausgewählt aus der Gruppe, bestehend aus mononukleären Leukozyten wie Lymphozyten und Monozyten, Granulozyten wie eosinophile und basophile Granulozyten, CD34⁺ hämatopoetischen Stammzellen, Leukämie- und Lymphomzellen und Zellen des Rhabdomyosarkoms und des malignen Melanoms.

Entsprechend einer weiteren Ausführungsform enthält die erfindungsgemäße Nukleinsäure Desoxyribonukleotide und/oder Ribonukleotide und/oder modifizierte Nukleotide wie beispielsweise Aminonukleotide, [α-S]-Trisphosphatnukleotide, Pseudouridinnukleotide und Nukleotide mit Basen, die chemische Modifikationen, beispielsweise Methyl-, Acetyl- oder Isopentenylgruppen, enthalten.

Gemäß einer weiteren Ausführungsform ist die erfindungsgemäße Nukleinsäure eine Antisense-Nukleinsäure, wobei die Nukleotidsequenz 5'-GTGGGAACATTCAACACTA-3' ist.

Der Begriff "Antisense-Nukleinsäure" bededeutet, daß mindestens ein Teil der Nuklotidsequenz der Nukleinsäure mindestens teilweise zu mindestens einem Teil der Nukleotidsequenz des Transkriptionsprodukts des für das Adhäsionsmolekül VLA-4 kodierenden Gens komplementär ist.

Eine weitere Ausführungsform der vorliegenden Erfindung betrifft einen Vektor, der mindestens eine wie oben definierte Nukleinsäure enthält. Der Begriff "Vektor" bedeutet ein DNA- und/oder RNA-Replicon, das zur Amplifikation einer oder mehrerer heterologer oder homologer Nukleotidsequenzen verwendet werden kann. Der erfindungsgemäße Vektor kann im Stand der Technik bekannte regulatorische Elemente enthalten. Insbesondere sind erfindungsgemäß DNA- oder RNA-Vektoren nützlich, welche die Transkription der erfindungsgemäßen Nukleinsäure ermöglichen. Ein Beispiel ist ein DNA-Vektor, der die erfindungsgemäße Nukleinsäure stromabwärts vom 3'-Ende eines geeigneten Promotors kodiert.

Eine weitere Ausführungsform der vorliegenden Erfindung betrifft eine Wirtszelle, die mindestens eine wie oben definierte erfindungsgemäße Nukleinsäure oder einen wie oben definierten erfindungsgemäßen Vektor enthält. Gemäß der vorliegenden Erfindung kann die Wirtszelle jede geeignete prokaryontische oder eukaryontische Zelle sein.

Eine weitere Ausführungsform der vorliegenden Erfindung betrifft eine pharmazeutische Zusammensetzung, welche die erfindungsgemäße Nukleinsäure oder den erfindungsgemäßen Vektor umfaßt, gegebenenfalls in Verbindung mit einem pharmazeutisch annehmbaren Lösungsmittel und/oderTräger.

Eine bevorzugte Ausführungsform der erfindungsgemäßen pharmazeutischen Zusammensetzung betrifft die Mobilisierung von Blutstammzellen in Säugern. Der Begriff "Mobilisierung von Blutstammzellen" bedeutet, daß die adhäsiven Interaktionen zwischen den Blutstammzellen wie beispielsweise CD34⁺ hämatopoetischen Stammzellen und der Mikroumgebung des Knochenmarks, umfassend die Stromazellen und die extrazelluläre Matrix, abgeschwächt bzw. aufgehoben werden, so daß die Blutstammzellen in die Gefäße des Säugers freigesetzt werden und dort zirkulieren.

Eine weitere bevorzugte Ausführungsform der erfindungsgemäßen pharmazeutischen Zusammensetzung betrifft die Behandlung maligner hämatologischer Erkrankungen in Säugern. Durch die Hemmung der adhäsiven Interaktionen zwischen malignen entarteten hämatopoetischen Zellen und der Mikroumgebung des Knochenmarks kommt es zur Hemmung der Proliferation der krankhaften Zellen. Vorzugsweise ist die maligne hämatologische Erkrankung ausgewählt aus der Gruppe akuter myeloischer Leukämien (AML), akuter lymphatischer Leukämien (ALL), chronischer myeloischer Leukämien (CML), chronischer lymphatischer Leukämien (CLL) und maligner Lymphome.

Eine weitere bevorzugte Ausführungsform der erfindungsgemäßen pharmazeutischen Zusammensetzung betrifft die Hemmung der Metastasierung von Tumorzellen, die VLA-4 exprimieren, in Säugern. Vorzugsweise sind die Tumorzellen, die VLA-4 exprimieren, Zellen des Rhabdomyosarkoms und/oder des malignen Melanoms.

Eine weitere bevorzugte Ausführungsform der erfindungsgemäßen pharmazeutischen Zusammensetzung betrifft die Behandlung von pathologischen Entzündungsreaktionen in Säugern. Vorzugsweise ist die pathologische Entzündungsreaktion ausgewählt aus der Gruppe akuter oder chronischer entzündlicher Erkrankungen bestehend aus chronisch-entzündlichen Darmerkrankungen wie beispielsweise M. Crohn und Colitis Ulcerosa, rheumatischen Systemerkrankungen wie beispielsweise rheumatoide Arthritis und Lupus erythematodes, Multiple Sklerose, Autoimmunerkrankungen wie beispielsweise Glomerulonephritis, entzündlichen Hauterkrankungen wie beispielsweise Neurodermitis, Psoriasis und Akne, "Graft-versus-host"-Reaktionen nach allogener Knochenmark- oder Blutstammzelltransplantation, Organabstoßungsreaktionen nach Transplantation, Allergien, Asthma, Virusinfektionen und Immunreaktionen bei wiederholter Applikation von Virusvektoren, wie beispielsweise Adenovirusvektoren am Bronchialepithel, in der Gentherapie.

Vorzugsweise ist der Säuger bei den bevorzugten Ausführungsformen der wie oben definierten pharmazeutischen Zusammensetzung *Homo sapiens sapiens* (Mensch).

Eine Anwendung der erfindungsgemäßen Nukleinsäure, des erfindungsgemäßen Vektors, der erfindungsgemäßen Wirtszelle oder der erfindungsgemäßen pharmazeutischen Zusammensetzung betrifft beispielsweise ein Verfahren zur Inhibierung der Expression des Adhäsionsmoleküls VLA-4 in Säugern, umfassend das Einbringen der erfindungsgemäßen Nukleinsäure, des erfindungsgemäßen Vektors, der erfindungsgemäßen Wirtszelle oder der erfindungsgemäßen pharmazeutischen Zusammensetzung in den Säugerorganismus. Das Verfahren kann beispielsweise zur Mobilisierung von Blustammzellen wie CD34⁺-Zellen in Säugern wie oben definiert, zur Behandlung wie oben definierter pathologischer Entzündungsreaktionen, zur Behandlung wie oben definierter maligner hämatologischer Erkrankungen oder zur Hemmung der Metastasierung wie oben definierter solider Tumoren_eingesetzt werden.

Der Begriff "Säugerorganismus" umfaßt jegliche Arten von Säugern gewonnener Einzelzellen, beispielsweise Kulturzellen als auch den Säuger *per se.*

Der Begriff "Einbringen" in einen Säugerorganismus kann im Fall von Einzelzellen, wie z.B. Kulturzellen von Säugerzellinien eine chemische Transfektion, z.B. mittels der Ca-Phosphat-Methode oder mittels der Lipofektion, oder eine Elektroporation, beim gesamten Tier bzw. Menschen eine subkutane, intramuskuläre, orale, peritoneale oder intravenöse Verabreichung der erfindungsgemäßen Nukleinsäure, des erfindungsgemäßen Vektors, der erfindungsgemäßen Wirtszelle oder der erfindungsgemäßen pharmazeutischen Zusammensetzung umfassen. Eine intravenöse Verabreichung kann beispielsweise eine Bolusinjektion und/oder eine kontinuierliche Infusion umfassen.

Die Figuren zeigen:

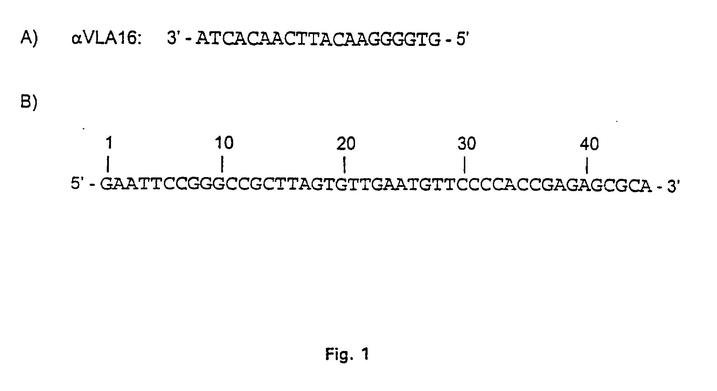

- Fig. 1: (A) Nukleotidsequenz einer erfindungsgemäßen Nukleinsäure (Oligodesoxyribonukleotid (ODN) αVLA16), gerichtet gegen die mRNA der α4-Kette von VLA-4. (B) Lokale Zielnukleotidsequenz der mRNA der α4-Kette von VLA-4 (Positionen 1 bis 45; Takada et al., 1989).

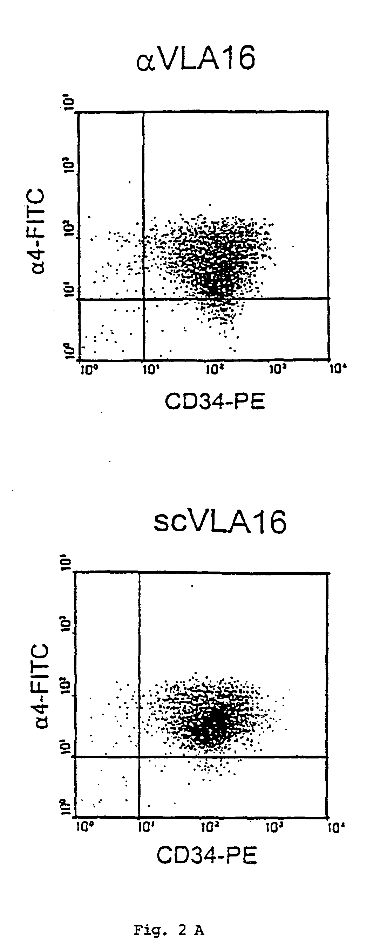

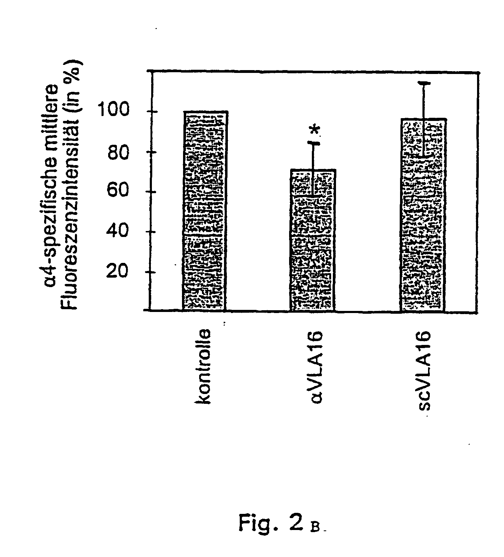

- Fig.2: Graphische Darstellung der Inhibierung der VLA-4-Expression auf CD34⁺-Zellen durch das erfindungsgemäße ODN αVLA16. Hämatopoetische CD34⁺-Stammzellen wurden über drei Tage täglich mit den angegebenen ODN bzw. mit der Kontrolle transfiziert und am vierten Tag analysiert. Die VLA-4-Expression wurde durch eine Zwei-Farben-Immunfluoreszenzanalyse [monoklonale Antikörper (mAk) CD34⁺ und CD49D] durchflußzytometrisch bestimmt. (A) Durchflußzytometrische Analyse der CD34- und der VLA-4-Expression nach Transfektion des erfindungsgemäßen ODN αVLA16 im Vergleich zum Kontroll-ODN scVLA16 eines repräsentativen Experiments. (B) VLA-4-spezifische mittlere FITC-Fluoreszenzintensität auf CD34⁺-Zellen nach Transfektion von ODN in 6 unabhängigen Experimenten. Nach Transfektion des erfindungsgemäßen Antisense-ODN αVLA16 wurde im Vergleich zum Kontroll-ODN scVLA16 eine signifikante (t-Test für gepaarte Proben) Verringerung der VLA-4-Expression von 29% (SD: 13,1%; p < 0,01) beobachtet.

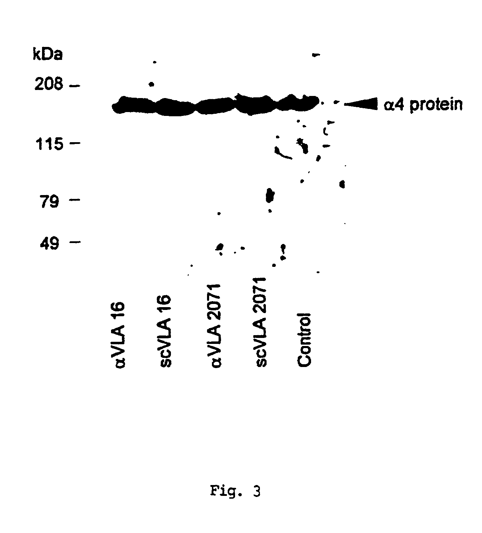

- Fig. 3: Photographische Darstellung zum Nachweis der Inhibierung der VLA-4-Expression auf CD34⁺-Zellen durch das erfindungsgemäße ODN αVLA16 mittels Western-Blot-Analyse. Die densitometrisch gemessene Intensität der 150 kDa α4-Proteinbande (s. Pfeilspitze) von VLA-4 war bei CD34⁺-Zellen, die mit dem erfindungsgemäßen ODN αVLA16 transfiziert wurden, um 35% vermindert.

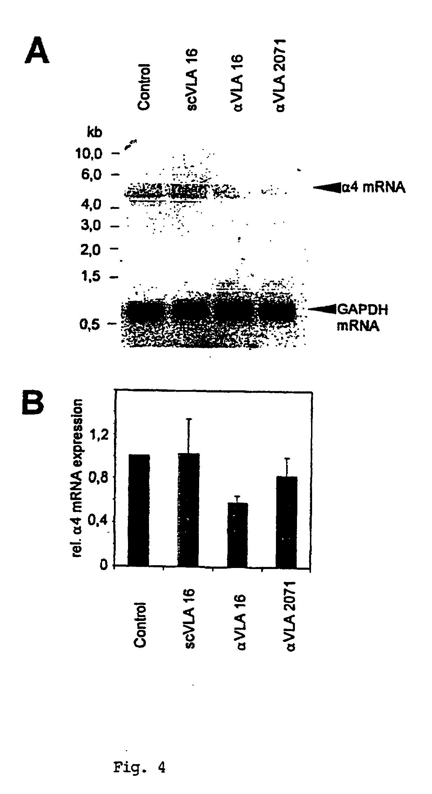

- Fig. 4: Nachweis der inhibierung der VLA-4-Expression in CD34⁺-Zellen auf mRNA-Ebene durch das erfindungsgemäße ODN αVLA16 mittels Northern-Blot-Analyse. (A) Photographische Darstellung einer repräsentativen Northern-Blot-Analyse von CD34⁺-Zellen, die mit den ODN αVLA16 und αVLA2071 bzw. einem Kontroll-ODN transfiziert wurden. Durch Pfeilspitzen angezeigt sind die Banden der α4-mRNA von VLA-4 und der GAPDH-mRNA, die als Referenzbande zur Standardisierung bei der Quantifizierung verwendet wurde. (B) Graphische Darstellung der quantitativen Auswertung der Bandenintensitäten der Northern-Blot-Analyse durch einen Phosphorlmager. Gezeigt ist das Ergebnis aus zwei unabhängigen Experimenten. Eine spezifische Verminderung der α4-mRNA-Bande von 42% (SD: 6,5%) wurde nur beim erfindungsgemäßen ODN αVLA16 beobachtet.

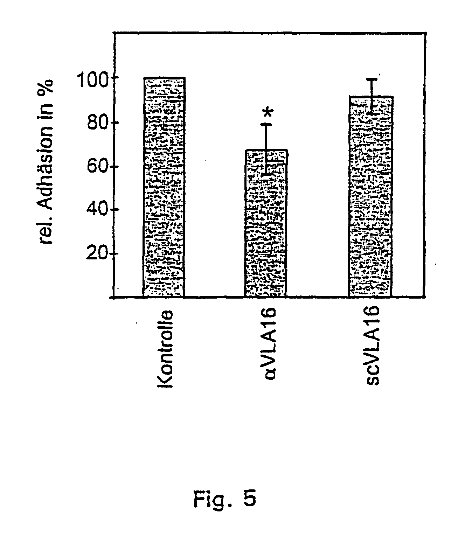

- Fig. 5: Graphische Darstellung zur Wirkung der Inhibierung der VLA-4-Expression auf die Adhäsion von CD34⁺-Zellen auf Endothelzellen. Die Adhäsionseigenschaften der transfizierten CD34⁺-Zellen wurden auf IL-1β-stimulierten Endothelzellen (Endothelzelllinie ECV304) gemessen. Dazu wurden die CD34⁺-Zellen vor dem Adhäsionstest mit dem Fluoreszenzfarbstoff Calcein angefärbt und der Anteil adhärenter Zellen durch die Fluoreszenzintensität bestimmt. Nach der Transfektion mit αVLA16 wurde eine signifikante Reduzierung des Anteils adhärenter CD34⁺-Zellen von 29% (SD: 19%; p<0,05) gefunden.

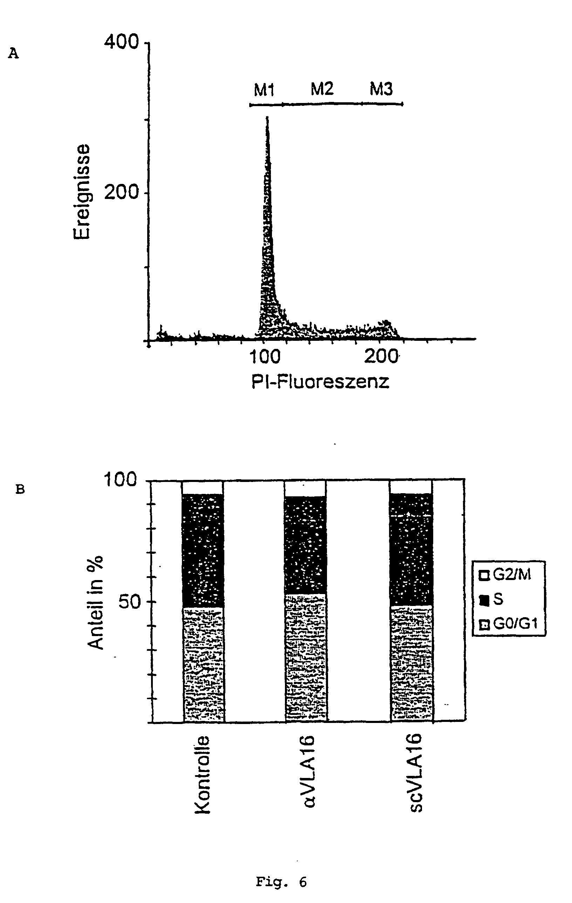

- Fig. 6: Graphische Darstellung zur Wirkung der Inhibierung der VLA-4-Expression auf die Proliferation von CD34⁺-Zellen. Nach der Transfektion mit dem erfindungsgemäßen Antisense-ODN αVLA16 bzw. mit der Kontrolle wurden die Zellen gezählt und einer Zellzyklusanalyse unterworfen. Nach vier Tagen in Suspensionskultur in einem Medium mit einem Cytokin-Cocktail wurden die Zellen expandiert, jedoch wurden keine Unterschiede in den Zellzahlen der mit dem erfindungsgemäßen Antisense-ODN behandelten Proben im Vergleich zu den mit dem Kontroll-ODN behandelten Proben beobachtet. (A) Durchflußzytometrische Zellzyklusanalyse von CD34⁺-Zellen nach 4 Tagen in Suspensionskultur eines repräsentativen Experiments. Die Region M1 wurde als G0/G1-Phase, die Region M2 als S-Phase und die Region M3 als G2/M-Phase gewertet. (B) Graphische Darstellung der Verteilung der αVLA16-transfizierten CD34⁺-Zellen über die einzelnen Zellzyklusphasen im Vergleich zu den Kontrollen. Nach Überprüfung der Antisense-vermittelten inhibierung der VLA-4-Expression der CD34⁺-Zellproben hatte sich der Anteil der Zellen in den G2/M- und S-Phasen nicht geändert, was darauf hinweist, daß der Grad der VLA-4-Expression keinen Einfluß auf die Proliferation von CD34⁺-Zellen in Suspensionskultur hat.

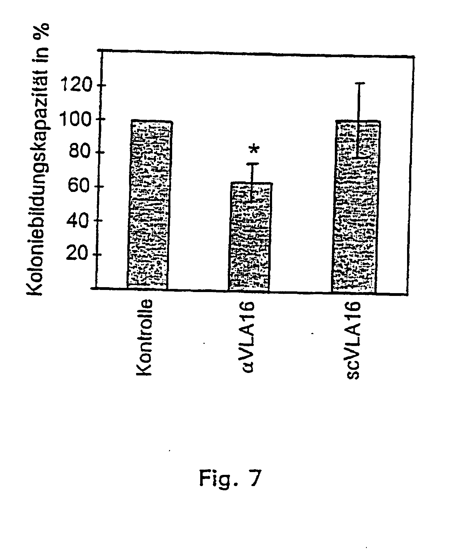

- Fig. 7: Graphische Darstellung der Koloniebildungskapazität von CD34⁺-Vorläuferzellen mit reduzierter VLA-4-Expression. Nach der Transfektion mit den angegebenen ODN bzw. mit der Kontrolle wurde die Koloniebildungskapazität von CD34⁺-Vorläuferzellen unter Verwendung eines semisoliden Kulturverfahrens untersucht. Anhand der Koloniezahlen CFU-GM und BFU-E wurde eine signifikante Inhibierung der Koloniebildungseffizienz von 39% (SD: 12,8%; p<0,01) nach der Transfektion mit dem erfindungsgemäßen VLA-4-spezifischen Antisense-ODN gefunden. Die Zahlen beider Kolonietypen wurden in ähnlicher Weise verringert, was darauf hinweist, daß keine bevorzugte Inhibierung einer bestimmten hämatopoetischen Linie erfolgt.

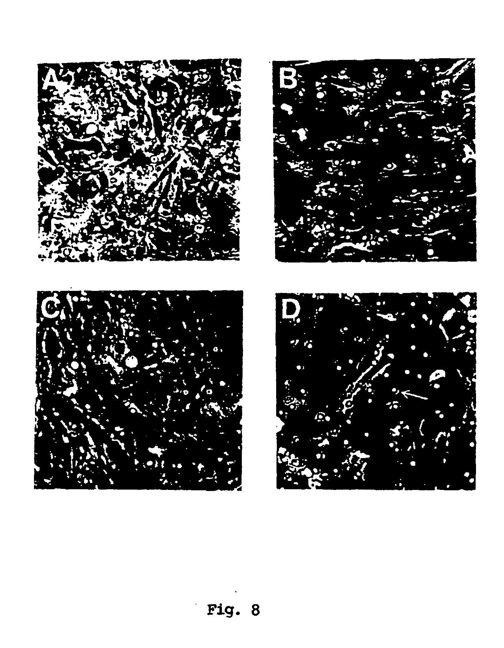

- Fig. 8: Photographische Darstellung, die den Einfluß von VLA-4-gerichteten Antisense-ODN auf die Morphologie von "Dexter-Typ"-Langzeitkulturzellen zeigt. Knochenmarklangzeitkulturen wurden vom Knochenmark gesunder, freiwilliger Spender angelegt. Nach sechs Wochen wurden ODN mit dem Transfektionsreagenz DOTAP in die Zellen der sich bis dahin ausgebildeten Stromaschicht transfiziert. Nach drei Tagen wurde die Morphologie der Zellen mikroskopisch untersucht. (A) Langzeitkultur vor Transfektion mit dem erfindungsgemäßen Antisense-ODN αVLA16, (B) Langzeitkultur vor Transfektion mit dem Kontroll-ODN scVLA16, (C) Langzeitkultur nach Transfektion mit αVLA16, (D) Langzeitkultur nach Transfektion mit scVLA16. Beim Auszählen der adhärenten mononukleären Zellen (weißer Pfeil) in einem definierten Gesichtsfeld von 25 cm² bei einer 100-fachen Vergrößerung fand sich in den mit dem erfindungsgemäßen Antisense-ODN behandelten 5 Langzeitkulturen von insgesamt 3 Spendern eine im Durchschnitt etwa 50% kleinere Zahl an mononukleären Zellen auf dem Stroma im Vergleich zu den mit dem Kontroll-ODN behandelten Kulturen und im Vergleich zu den Kulturen vor der Behandlung.

Die vorliegende Erfindung wird durch das folgende nicht-einschränkende Beispiel erläutert.

### BEISPIEL

### Zellen

Humane mononukleäre Zellen aus dem Blut wurden von Patienten (mit malignen hämatologischen Erkrankungen oder soliden Tumoren, die sich in kompletter Remission befanden) nach ihrer Einverständniserklärung erhalten. Die Zellen wurden durch Leukapherese unter Verwendung eines Fenwal CS3000 (Baxter Deutschland, München) nach cytotoxischer Chemotherapie, unterstützt durch rekombinantes humanes G-CSF (R-metHug-CSF; Amgen, Thousends Oaks, Kalifornien, USA), erhalten. Die Anreicherung der CD34⁺-Zellen aus den Leukaphereseprodukten wurde entweder mit dem "miniMACS"immunmagnetischen Trennungssystem (Miltenyi Biotec, Bergisch Gladbach) oder mit dem Isolex 300 SA Magnetzelltrennungssystem (Baxter Deutschland) entsprechend den Anleitungen der Hersteller wie zuvor beschrieben (Deichmann et al., 1997; Hohaus et al., 1997) durchgeführt. Nach der Gewinnung wurden die CD34⁺-Zellen in RPMI-1640-Medium (Sigma, Deissenhofen), supplementiert mit 10% hitzeinaktiviertem fötalem Kälberserum (FCS) (Boehringer Ingelheim Bioproducts, Heidelberg), 100 IU/mL Penicillin (Life Technologies, Karlsruhe), 100 µg/mL Streptomycin (Life Technologies), 2 mmol/L L-Glutamin (Life Technologies), 100ng/mL Interleukin-3 (IL-3), 10ng/mL Stammzellfaktor (SCF), 100ng/mL IL-6 und 3ng/mL IL-1β, gezüchtet. Die Cytokine wurden von PromoCell (Heidelberg) erhalten.

Die Endotheliale Zelllinie ECV304 (ATCC, Rockville, Maryland, USA), die von spontan transformierten, humanen endothelialen Zellen der Umbilikalvene (Takahashi et al., 1990) abgeleitet ist, wurde in Medium 199 (Life Technologies), supplementiert mit 10% FCS, L-Glutamin (2mmol/L), Penicillin (100IU/ml) und Streptomycin (100 mg/mL), gezüchtet und zweimal pro Woche nach Trypsinisierung passagiert.

### Klonogener Test hämatopoetischer Vorläuferzellen

Die Menge hämatopoetischer Vorläuferzellen wurde unter Verwendung eines semisoliden Zellkulturverfahrens beurteilt. Die Zellen wurden in Methylzellulosewachstumsmedium (MethoCult4433; StemCell Technology, Vancouver, Kanada) in einer Zelldichte von 1 x 10³/mL bei CD34⁺-Zellen oder 3 x 10⁵/mL bei Zellen aus dem Knochenmarkslangzeitkulturüberstand angesetzt. Die Koloniezahlen [Granulozyten/Macrophagen-bildende Kolonie (CFU-GM); Erythrozyten-bildende Kolonie (BFU-E)] wurden nach zwei Wochen gezählt. Die Klonierungseffizienz der Zellen wurde als die Zahl koloniebildender Zellen pro 1 x 10⁵ eingesetzter Zellen bestimmt.

### Knochenmarkslangzeitkultur (LTBMC)

Knochenmarkszellen wurden von gesunden Freiwilligen nach ihrer Einverständniserklärung durch Beckenkammpunktion erhalten. Eine LTBMC myeloider Zellen wurde mit Veränderungen wie zuvor beschrieben (Dexter et al., 1977; Miyake et al., 1991) durchgeführt. Dazu wurden mononukleäre Zellen durch Dichtezentrifugation unter Verwendung des Lymphozytentrennungsmediums Lymphoprep (Nycomed Pharma, Oslo, Norwegen) getrennt. 2 x 10⁷ mononukleäre Zellen wurden in einer 25 cm² Zellkulturflasche in 10 mL MyeloCult-Medium (Stem Cell Technologies, Vancouver, Kanada), supplementiert mit 10⁻⁷M Hydrocortisonnatriumsalz (Sigma, Deissenhofen) gezüchtet. Die Zellen wurden in 5% CO₂ bei 33°C inkubiert. Die Hälfte des Mediums wurde wöchentlich durch frisch hergestelltes, Hydrocortison enthaltendes Medium ersetzt. Nach sechs Wochen wurden die LTBMC für Transfektionsexperimente und für Analysen des Zellkulturüberstandes verwendet.

### Oligodesoxyribonukleotidsynthese und Transfektion

Es wurden 20mere Phosphorthioat-ODN verwendet. Die ODN hatten die folgenden Sequenzen:

αVLA16:   5'-GTGGGGAACATTCAACACTA-3'

scVLA16: 5'-TAGCTAGTAGACTGCAAGAC-3'

Das Kontroll-ODN scVLA weist eine zufällig zusammengesetzte Nukleotidsequenz aus den Nukleotiden des erfindungsgemäßen Antisense-ODN αVLA16 auf. Die ODN wurden durch Standard-Phosphoramidatchemie bei Interactiva (Ulm) synthetisiert und wurden nach der Synthese vom Hersteller durch Umkehrphasenhochleistungsflüssigkeitschromatographie gereinigt und anschließend lyophilisiert. Vor der Verwendung wurden die ODN in Hepes-Puffer (20 mM, pH 7,4) gelöst.

Die Transfektion von CD34⁺-Zellen wurde mit dem kationischen Lipid DOTAP (Boehringer Mannheim, Mannheim) wie zuvor beschrieben (Kronenwett et al., 1998) durchgeführt. Kurz beschrieben wurden die Zellen in einem Endvolumen von 1 mL bei einer Konzentration von 2 x 10⁶-Zellen/mL inkubiert. Vor der Transfektion wurden die ODN (Endkonzentration in der Zellsuspension: 0,5 µM) in Hepes-Puffer (20 mM, pH 7,4) mit DOTAP bei einem Verhältnis von ODN/DOTAP von 1:5 (w:w) für 15 min bei Raumtemperatur inkubiert. Nach dem Hinzufügen der ODN/DOTAP-Komplexe zu den Zellen wurden die Proben für 24 Stunden bei 37°C inkubiert. Die Transfektion wurde täglich in den folgenden zwei Tagen mit der Hälfte der Dosierung an ODN und DOTAP wiederholt. 24 Stunden nach der letzten Transfektion wurden die Zellen der fluoreszenzaktivierten Zellsortierung (FACS) oder funktionellen Tests unterworfen.

Zur Transfektion einer LTBMC wurde die Hälfte des Mediums (5 mL) durch frisches Medium ersetzt, und die vorinkubierten ODN/DOTAP-Komplexe wurden in einer Endkonzentration von 0,5 µM hinzugefügt. Zwei weitere Transfektionen wurden während der beiden darauffolgenden Tage durchgeführt, und die Überstände der LTBMC wurde den weiteren Analysen einen Tag nach der letzten Transfektion unterworfen.

### Immunfluoreszenzfärbung und Durchflußzytometrie

Nach dem Waschen mit phosphatgepufferter Salzlösung (PBS) wurden 1 x 10⁵-Zellen mit den Fluoresceinisothiocyanat-(FITC-) oder Phycoerythrin-(PE-) gekoppelten monoklonalen Antikörpern (mAk) in 500 mL PBS, enthaltend 1% BSA, bei 4°C für 30 min inkubiert. Die folgenden mAk wurden verwendet: CD34-PE (Klon HPCA-2; Becton-Dickinson, Heidelberg) und CD49d-FITC (Klon HP2/1; Immunotech, Marseille, Frankreich). Isotypidentische mAk dienten als Kontrolle (FITC/PE-gekoppelte IgG1; Becton-Dickinson). Nach der Antikörperfärbung wurden die Zellen gewaschen und in PBS-Puffer suspendiert. Die Zellen wurden mit einem FACScan von Becton-Dickinson (Heidelberg) mit einem 2-W Argonionenlaser analysiert. Die Fluoreszenz wurde mit 530/15 nm (FITC) und 575/36 nm (PE) Banddurchlaßfiltern gemessen. Die Daten wurden mit der CELL QUEST-Software von Becton-Dickinson, nachdem der Auswertungsbereich auf lebende Zellen festgelegt wurde, analysiert. Zur Beurteilung der VLA-4-Expression auf CD34⁺-Zellen wurde die Zwei-Farben-Immunfluoreszenzanalyse verwendet. Nach dem Festlegen des Auswertungsbereichs auf CD34⁺-Zellen wurde die durchschnittliche Fluoreszenzintensität von VLA-4 (α4-Kette, CD49d) durch die Software berechnet und in relativen Einheiten ausgedrückt.

### Zellzyklusanalyse

Zur Zellzyklusanalyse wurden 5 x 10⁵-Zellen mit PBS gewaschen, in einer Lösung, enthaltend 500 µL FCS, 500 µL RPMI 1640-Medium und 3 mL eiskalten Ethanol, resuspendiert und auf Eis für 30 min inkubiert. Danach wurden die Zellen zentrifugiert und in einer Lösung, enthaltend 450 µL Propidiumiodid (50µg/mL; Sigma, Deissenhofen) und 50 µL RNAse H (5 mg/mL; Sigma), resuspendiert. Nach der Inkubation auf Eis für 30 min wurden die Zellen direkt der FACS-Analyse unterworfen. Nach dem Festlegen des Auswertungsbereichs auf mononukleäre Zellen, entsprechend der Vorwärts- und Seitwärtsstreuungscharakteristika, wurde die Propidiumiodidfluoreszenz gemessen. Der Anteil der Zellen in der G0/G1-, S- und G2/M-Phase des Zellzyklus wurde mit dem CELL FIT-Programm (Becton-Dickinson) berechnet.

### Western-Blot-Analyse

CD34⁺-Zellen wurden mit 80 µL Triton X-100 (1%) bei 4°C für 5 min lysiert. Unlösliche Zelltrümmer wurden durch Zentrifugation des Lysats bei 10.000xg für 10 min abgetrennt, und das Proteinextrakt im Überstand wurde zur Western-Blot-Analyse verwendet. 14 µg des Proteinextraktes wurden der denaturierenden SDS-Gelelektrophorese wie zuvor beschrieben unterworfen (Sambrook et al., 1989). Als Standard wurde ein vorgefärbter Standard mit einem breiten Molekulargewichtsbereich verwendet (BioRad, München). Die Proteine wurden auf eine Polyvinylidenfluorid-Membran mit einer Porengröße von 0,45 µm (Immobilon P, Millipore, Bedford, Massachusettes, USA) bei 0,8 mA/cm² in einem Puffer, enthaltend 25 mM Tris, 40 mM Amino-n-Capsinsäure und 20% Methanol, für 90 min übertragen. Nach der Elektroübertragung wurde die Membran in 1 x phosphatgepuffter Salzlösung (PBS), enthaltend 5% fettarme Trockenmilch, bei Raumtemperatur für eine Stunde inkubiert. Danach wurde die Membran mit der Bindungslösung (1 x PBS, 0,5% Triton-x-100, 0,5% fettarme Trockenmilch), enthaltend 1 µg/mL VLA-4 monoklonalen Antikörper (Klon HP2/1, Immunotech, Marseille, Frankreich) bei 4°C für 24 Stunden durchtränkt, 2 mal in der Bindungslösung für 5 min gewaschen und bei Raumtemperatur für zwei Stunden in der Bindungslösung, enthaltend Meerrettichperoxidase-gekoppelte Ziege-Anti-Maus-lg, inkubiert. Die Antikörperbindung wurde photochemisch, wie beschrieben (Leong et al., 1986), detektiert. Kurz beschrieben wurde die Membran nach dem viermaligen Waschen in der Bindungslösung mit einer Lösung, enthaltend 8 mL Luminol-Puffer (100 mM Tris-HCI pH 8,8, 1,25 mM Natriumluminol, 2,7 mM H₂O₂), 2 mL H₂O und 20 µL Verstärkerlösung (11 mg p-Hydroxycumarinsäure in 10 mL DMSO) für 1 min inkubiert und danach auf einem Röntgenfilm (Biomax, Kodak, Rochester, New York, USA) exponiert. Alle Chemikalien wurden von Sigma, (Deissenhofen) erhalten.

### Northern-Blot-Analyse

Die RNA von 5 x 10⁶-Zellen wurde mittels eines "RNeasy" RNA-Extraktionskits (Qiagen, Hagen) gemäß der Anleitung des Herstellers extrahiert. Die RNA wurde von den Säulen in 25 µL mit DEPC behandeltem, destilliertem Wasser eluiert. 10 µg RNA wurden der Agarosegelektrophorese unterworfen und auf eine Nitrozellulosemembran (Hybond-N, Amersham, Braunschweig) wie zuvor beschrieben (Kroczek, 1993) übertragen. Kurz beschrieben wurde die denaturierte RNA mit Formaldehyd-haltigen 1%-Agarosegelen in Gegenwart von Ethidiumbromid unter Verwendung von MOPS-Laufpuffer (0,02 M 4-Morpholinopropansulfonsäure, 5 mM Natriumacetat, 1 mM EDTA, pH 5,5 - 7,0) getrennt. Nach der Elektrophorese wurde die RNA über Nacht auf die Nitrozellulosemembran mit 10 x SSC (20 x SSC: 3 M NaCI, 0,3 M Na₃ Citrat, pH 7,0) übertragen. Zur Vernetzung wurden die Membranen UV-Licht ausgesetzt, und die Hybridisierung mit radioaktiv markierten Proben wurde wie beschrieben (Bellon et al,. 1994) durchgeführt. Kurz beschrieben wurde die Membran in einer Lösung, enthaltend 50% deionisiertes Formamid, 5 x SSC, 5 x Denhardt's-Lösung, 50 mM NaH₂PO₄/Na₂HPO₄ (pH 6,5), 250 µg/mL tRNA aus Bäckerhefe (Boehringer Mannheim, Mannheim), bei 42°C für 10 Stunden inkubiert. Danach wurden die Membranen in der gleichen Lösung mit der radioaktiv markierten Probe (10⁶ cpm/mL) bei 42°C für 16 Stunden hybridisiert, mit 2 x SSC/0,5% SDS bei Raumtemperatur für 15 min gewaschen und mit 0,3 x SSC/0,5% SDS bei 65°C für 15 min gewaschen. Alle Chemikalien wurden von Sigma (Deissenhofen) erhalten. Die Membranen wurden auf einem Röntgenfilm exponiert.

Die DNA-Proben wurden mit [α-³²P]-dCTP (Amersham, Braunschweig) unter Verwendung eines Markierungskits (Boehringer Mannheim, Mannheim) gemäß den Instruktionen der Hersteller markiert. Als spezifische Probe für die α4-mRNA wurde ein 3,8 kb SalI/XbaI-Fragment der α4-cDNA (Takada et al., 1989) verwendet, das von American Type Culture Collection (Manassas, Virginia, USA; Nr. 95495) erhalten wurde. Als interner Standard wurde die mRNA der Glycerinaldehyd-3-phosphatdehydrogenase (GAPDH) verwendet. Eine GAPDH-spezifische DNA-Probe wurde durch PCR-Amplifikation unter Verwendung einer cDNA, die aus RNA der leukämischen Zelllinie HL60 wie beschrieben (Deichmann et al. 1997) hergestellt wurde, erhalten. Die Nukleotidsequenzen der GAPDH-spezifischen Primer waren: 5'-TCCATGACAACTTTGGTATCG-3' und 5'-GTCGCTGTTGAAGTCAGAGGA-3'. Das sich ergebende PCR-Produkt wurde aus einem Agarosegel eluiert, radioaktiv markiert und zur Hybridisierunglösung hinzugefügt.

Zur Quanitifzierung des α4-mRNA-Spiegels, wurden die Membranen mit einem Phosphorlmager (Molecular Dynamics, Krefeld) abgetastet, und die Intensitäten der α4-Banden sowie der GAPDH-Banden wurden mit der IMAGE QUANT-Software (Molecular Dynamics) gemessen. Die Intensität jeder α4-Bande wurde auf die entsprechende GAPDH-Bande normalisiert. Ein Wert von 1 wurde für die normalisierte Intensität der α4-Bande von Proben aus Kontrollzellen bestimmt.

### Adhäsionstest

1,5 x 10⁴ ECV304-Zellen wurden pro Vertiefung in einer Gewebskulturschale mit 96 Vertiefungen angesetzt und bis zur Konfluenz gezüchtet. Zur Erhöhung der Expression von Adhäsionsmolekülen wurde die endotheliale Einzelschicht mit 200 U/mL rekombinantem, humanem Interleukin-1β (IL-1β; PromoCell, Heidelberg) 16 Stunden vor den Adhäsionsexperimenten inkubiert. Die stimulierten endothelialen Zellen wurden zweimal mit M199-Kulturmedium, enthaltend 1% RinderSerumalbumin (BSA; für 45 min bei 65°C hitzeinaktiviert), gewaschen. Danach wurden die Zellen mit dem BSA-haltigen Kulturmedium bei 37°C für eine Stunde abgesättigt. Währenddessen wurden transfizierte CD34⁺-Zellen mit dem Fluoreszenzfarbstoff Calcein unter Verwendung des Vybrant-Zelladhäsionskits (MoBiTec, Göttingen) gemäß der Anleitung des Herstellers markiert. 5 x 10⁶ markierte CD34⁺-Zellen wurden in 1 mL Kulturmedium, enthaltend 1% hitzeinaktiviertes BSA, resuspendiert, und 100 µL der CD34⁺-Zellsuspension, wurde zu einer Vertiefung mit den endothelialen Zelleinzelschichten hinzugefügt, und bis zu 10 Werte pro Probe wurden erhalten. Nach einer Koinkubationszeit von 30 min bei 37°C wurden nicht-adhärente Zellen durch dreimaliges sanftes Waschen mit Kulturmedium (1% BSA, hitzeinaktiviert) mit einer Mehrkanalpipette entfernt. Die Fluoreszenzintensität in jeder Vertiefung wurde mit einem VICTOR-Fluoreszenzdetektor (1420 Multilabel Counter, Fa. Wallac, Turko, Finnland) bei 535 nm gemessen. Vertiefungen mit endothelialen Zellen und unmarkierten CD34⁺-Zellen wurden zur Bestimmung der Hintergrundfluoreszenz verwendet. Vertiefungen mit endothelialen Zellen sowie markierten CD34⁺-Zellen, die nach der Koinkubation nicht gewaschen worden waren, wurden zur Berechnung der Fluoreszenzintensität sowohl adhärenter als auch nicht-adhärenter CD34⁺-Zellen verwendet. Der Anteil adhärenter CD34⁺-Zellen in jeder Vertiefung wurde als diejenige Fluoreszenzintensität der jeweiligen Vertiefung, dividiert durch die Fluoreszenzintensität der Vertiefung mit markierten adhärenten und nicht-adhärenten CD34⁺-Zellen, berechnet.

### Statistische Auswertung

Die Daten wurden als Mittelwerte angegeben und die Standardabweichung (SD) wurde berechnet. Die statistische Signifikanz wurde mit Hilfe des t-Tests für gepaarte Proben als Vergleich zwischen den mit dem erfindungsgemäßen Antisense-ODN αVLA16 und mit dem Kontroll-ODN scVLA16 behandelten Zellen bestimmt. Ein p-Wert kleiner als 0,05 wurde als statistisch signifikant angesehen.

### 1. Inhibierung der VLA-4-Expression auf CD34⁺-Zellen

Zur spezifischen Inhibierung der VLA-4-Expression auf CD34⁺-hämatopoetischen Stammzellen wurde ein synthetisches Antisense-ODN (αVLA16) eingesetzt, das gegen die mRNA der α4-Kette von VLA-4 gerichtet ist. Die spezifische biologische Aktivität eines Antisense-ODN hängt wesentlich von der Wahl einer geeigneten Sequenz innerhalb der Ziel-mRNA aufgrund von Sekundär- und Tertiärstrukturen als auch von funktionellen Elementen der mRNA ab. Das erfindungsgemäße Antisense-ODN αVLA16 ist gegen das Initiationskodon cAUG gerichtet. Zum Vergleich wurde ein ODN (scVLA16) eingesetzt, das eine zufällig zusammengesetzte Nukleotidsequenz aus den Nukleotiden des erfindungsgemäßen Antisense-ODN αVLA16 aufweist.

Die Nukleotidsequenz des erfindungsgemäßen Antisense-ODN αVLA16 ist zusammen mit der lokalen Zielnukleotidsequenz der mRNA der α-Kette von VLA-4 (Positionen 1 bis 45) in der Fig. 1 gezeigt.

CD34⁺ hämatopoetische Stammzellen von Leukaphereseprodukten wurden durch immunmagnetische Selektion angereichert. Zwischen 85% und 95% der CD34⁺-Zellen waren sofort nach der Reinigung VLA-4-positiv. Zur Untersuchung des Grades der Expression von VLA-4 während der Suspensionskultur von CD34⁺-Zellen in einem Transfektionsmedium, enthaltend die Cytokine IL-3, IL-6, SCF und IL-1β, wurde die durchschnittliche Intensität der VLA-4-spezifischen Fluoreszenz an den Tagen 0, 1 und 4 der Kultur gemessen. Die Werte wurden durch Division der durchschnittlichen VLA-4-spezifischen Fluoreszenzintensität durch die Fluoreszenzintensität des isotypischen Kontrollantikörpers standardisiert. Der Quotient war 5 nach der immunmagnetischen Selektion am Tag 0, 7 nach 24 Stunden in Suspensionskultur in Gegenwart der Cytokine und 8,1 nach vier Tagen, was einen 1,4-fachen Anstieg der VLA-4-Expression während der ersten 24 Stunden der *Ex-vivo*-Kultur anzeigt. Zur Transfektion der ODN in isolierte CD34⁺-Zellen wurde das kationische Lipid DOTAP verwendet, was die zelluläre Aufnahme von ODN in primäre Leukozyten wie CD34⁺-Zellen erleichtert (Kronenwett et al., 1998). Die Antisense-ODN-vermittelte Verminderung der Expression von VLA-4 auf der Oberfläche wurde anhand einer Zwei-Farben-lmmunfluoreszenzanalyse nach Festlegung des Auswertebereichs auf CD34⁺-Zellen beurteilt. Die CD34⁺-Zellen wurden über drei Tage täglich transfiziert und am vierten Tag analysiert. Eine signifikante Verringerung der durchschnittlichen VLA-4-Fluoreszenzintensität von 29% (SD: 13,1%) wurde bei Verwendung des erfindungsgemäßen Antisense-ODN αVLA16 beobachtet, während kein signifikanter Effekt bei Verwendung des Kontroll-ODN gefunden wurde (Fig.2).

Das 51kDa große α4-Protein kann variabel an einer spezifischen Stelle gespalten werden, was zwei Fragmente mit 80 kDa und 70 kDa ergibt, die miteinander assoziiert bleiben (Hemler et al., 1987). In Abhängigkeit vom Zelltypus kommt α4 entweder ganz in der intakten 150 kDa großen Form, teilweise oder fast vollständig in die 80 kDa und 70 kDa großen Fragmente gespalten, vor. Um zu beurteilen, in welcher Form α4 in CD34⁺-Zellen vorliegt, als auch um auszuwerten, ob alle Formen in wesentlicher Weise durch Antisense-ODN inhibiert werden, wurde eine Western-Blot-Analyse von Proteinextrakten transfizierter CD34⁺-Zellen durchgeführt. Es konnte nur die intakte 150 kDa große Form des α4-Proteins in CD34⁺-Zellen nachgewiesen werden (Fig. 3). Übereinstimmend mit den Immunfluoreszenzdaten war die Bandenintensität der α4-Kette in der mit dem erfindungsgemäßen Antisense-ODN αVLA16 behandelten Probe im Vergleich mit dem Kontroll-ODN vermindert (Fig. 3).

Zur Beurteilung der Auswirkungen von Antisense-ODN auf den Spiegel der mRNA wurde eine Northern-Blot-Analyse transfizierter CD34⁺-Zellen unter Verwendung des ODN αVLA16 sowie des diesbezüglichen Kontroll-ODN durchgeführt. Eine spezifische Verminderung der beiden α4-mRNA-Banden wurde 48 Stunden nach der Transfektion mit αVLA16 beobachtet, während keine Inhibierung unter Verwendung des Kontroll-ODN gefunden wurde (Fig. 4A). Anhand der Beurteilung durch eine Phosphorlmager-Analyse war die Absenkung des α4-mRNA-Spiegels 42% (SD: 6,5%), wobei die mRNA von GAPDH als Bezugstranskript verwendet wurde (Fig. 4B).

Entsprechend dieser Ergebnisse wurde das erfindungsgemäße Antisense-ODN αVLA16 und das entsprechende Kontroll-ODN scVLA16 in weiteren funktionellen Experimenten verwendet.

### 2. Auswirkungen der Verminderung der VLA-4-Expression auf die Adhäsion und Proliferation von CD34⁺-Zellen

Der Einfluß der Verminderung der VLA-4-Expression auf die adhesiven Eigenschaften von CD34⁺-Zellen an eine mit IL-1β stimulierte endotheliale Zellschicht wurde nach der Markierung transfizierter CD34⁺-Zellen mit dem Fluoreszenzfarbstoff Calcein beurteilt. Eine signifikante Verminderung des Anteils adhärenter CD34⁺-Zellen von 29% (SD: 19%) wurde nach der Transfektion mit dem erfindungsgemäßen Antisense-ODN αVLA16 im Vergleich zum Kontroll-ODN gefunden (Fig. 5).

Nach der Transfektion mit Antisense-ODN wurden die Zellen durch Trypanblau-Ausschluß gezählt und zur Beurteilung der Auswirkungen der Verminderung der VLA-4-Expression auf die Proliferation einer Zellzyklusanalyse unterworfen. Nach fünf Tagen Suspensionskultur in einem Medium, enthaltend einen Cytokin-Cocktail aus SCF, IL-3, IL-6 und IL-1β wurden die Zellen dreifach expandiert, jedoch wurden keine Unterschiede in den Zellzahlen bei einem Vergleich der mit dem erfindungsgemäßen Antisense-ODN behandelten Zellen mit den Zellen, die mit dem Kontroll-ODN behandelt wurden, festgestellt. Bei der Zellzyklusanalyse waren 47,7% der unbehandelten Zellen in der G0/G1-Phase, 46,8% in der S-Phase und 5,6% in der G2/M-Phase. Bei der Beurteilung von CD34⁺-Zellproben mit Antisense-vermittelter Verminderung der VLA-4-Expression war der Anteil der Zellen in der G2/S-Phase nicht verändert, was darauf hinweist, daß die VLA-4-Expression keinen Einfluß auf die Proliferation von CD34⁺-Zellen in Suspensionskultur hat (Fig. 6).

Des weiteren wurde die Koloniebildungskapazität von CD34⁺-Vorläuferzellen mit verminderter VLA-4-Expression unter Verwendung eines semisoliden Kulturverfahrens nach der Transfektion mit ODN untersucht. Die Bestimmung von CFU-GM und BFU-E ergab eine signifikante Inhibierung der Koloniebildungseffizienz von 39% (SD: 12,8%) nach der Transfektion mit dem erfindungsgemäßen VLA-4-spezifischen Antisense-ODN (Fig. 7). Beide Kolonietypen waren in ähnlicher Weise vermindert, was darauf hinweist, daß keine bevorzugte Inhibierung einer bestimmten hämatopoetischen Linie vorliegt.

### 3. Mobilisierung von CD34⁺-Zellen in vitro nach Transfektion mit einem erfindungsgemäßen VLA-4-spezifischen Antisense-ODN

Zur Untersuchung des Einflusses der Antisense-ODN-vermittelten Verminderung der VLA-4-Expression bei CD34⁺-Zellen auf die Mobilisierung hämatopoetischer Vorläuferzellen wurden "Dexter-Typ"-Knochenmarkslangzeitkulturen als *In-vitro*-Modell der Stammzellmobilisierung verwendet. Sechs Wochen nach Beginn der Langzeitkultur hatte sich eine Stroma-Schicht entwickelt, die während drei aufeinanderfolgenden Tagen entweder mit αVLA16 oder mit dem Kontroll-ODN scVLA16 unter Verwendung von DOTAP als Transfektionsreagenz behandelt wurde. Morphologisch wurde eine um 50% verminderte Anzahl mononukleärer Zellen auf der Stromaschicht beobachtet, was semiquantitativ durch Zählung der Nicht-Stromazellen in einem Beobachtungsfeld von 25 cm² abgeschätzt wurde (Fig. 8; Tabelle 1). Die verminderte Anzahl adhärenter Zellen war von einer zweifach größeren Anzahl von Zellen im Überstand der Knochenmarkskultur, die durch Trypanblau-Aussschluß bestimmt wurde, begleitet (Tabelle 1). Mittels einer Immunfluoreszenzanalyse der Zellen im Überstand der Knochenmarkskulturen wurde eine Population von 0,77% CD34⁺-Zellen in den Kulturen, die mit dem erfindungsgemäßen Antisense-ODN behandelt wurden, festgestellt, während keine CD34⁺-Zellen in den Kulturen nachweisbar waren, die mit dem Kontroll-ODN behandelt wurden (Tabelle 1). Entsprechend dem Anstieg der Anzahl von CD34⁺-Zellen im Überstand der Langzeitkulturen war die Koloniebildungskapazität der Zellen aus den Überständen von Kulturen, die mit αVLA16 behandelt wurden, 30fach im Vergleich zu Kontrollproben erhöht (Tabelle 1). Dies weist auf eine Mobilisierung hämatopoetischer Vorläuferzellen aus dem Stroma in den Zellkulturüberstand bei Verwendung des erfindungsgemäßen VLA-4-spezifischen Antisense-ODN hin.

**Tabelle 1**

| | | | |
|---|---|---|---|
| Einfluß des erfindungsgemäßen Antisense-ODN αVLA16 auf die Mobilisierung von CD34⁺ hämatopoetischen Vorläuferzellen in "Dexter-Typ"-Knochenmarklangzeitkulturen. Drei Tage nach der Transfektion von Knochenmarklangzeitkulturen mit den angegebenen ODN wurde die Zahl adhärenter Zellen in einem Gesichtsfeld von 25 cm² bei einer 100-fachen Vergrößerung (vgl. Fig. 8) bestimmt sowie die nicht-adhärenten Zellen im Überstand auf die Zellzahl, auf den Anteil an CD34⁺ Zellen und auf den Anteil an Vorläuferzellen (koloniebildende Zellen im semisoliden Kulturverfahren) untersucht. Angegeben sind die Ergebnisse aus jeweils 5 Langzeitkulturen von 3 verschiedenen Knochenmarkspendern. Eine Population von 0,77% CD34⁺-Zellen und eine 30fache Erhöhung der Zahl koloniebildender Vorläuferzellen wurden in den Kulturen, die mit dem erfindungsgemäßen Antisense-ODN αVLA16 behandelt wurden, nachgewiesen. Im Vergleich zum Kontroll-ODN signifikant veränderte Werte (p<0,05) sind in fetter Schrift dargestellt. | | | |

| Parameter | vor Transfektion | αVLA16 | scVLA16 |
|---|---|---|---|
| nicht-adhärente Zellen im Überstand (pro µl) | 42,8 (SD: 12,5) | **96 (SD: 49,7)** | 37 (SD: 22) |
| | | | |
| adhärente mononukleäre Zellen (pro 25 cm²) | 49 (SD: 17,7) | **21,6 (SD: 6,7)** | 54,6(SD: 12,5) |
| | | | |
| Anteil CD34⁺-Zellen im Überstand (in %) | 0,14 (SD: 0,08) | **0,77 (SD: 0,69)** | 0,18 (SD: 0,2) |
| | | | |
| Koloniebildungseffizienz der Zellen aus dem Überstand (pro 1 x 10⁶ eingesetzter Zellen) | n.d. | **646 (SD: 411,8)** | 22 (SD: 16,4) |
| n.d.: nicht bestimmt | | | |

### REFERENZEN

Bellon et al. (1994) European Journal of Immunology 24, 41-47.

Chisholm et al. (1993) European Journal of Immunology 23, 682-688.

Craddock et al. (1997) Blood 90, 4779-4788.

Deichmann et al. (1997) Blood 89, 3522-3528.

Dexter et al. (1977) Journal of Cell Physiology 91, 335-339.

Ferguson et al. (1991) Proceedings of the National Academy of Science of the U.S.A. 88,8072-8076.

Haas et al., 1995, Blood 85,1986-1943.

Hemler et al. (1987) Journal of Biological Chemistry 262, 11478-11485.

Henes et al., 1994, Proceedings of the National Academy of Sciences of the U.S.A. 91, 5187-5191.

Hohaus et al. (1997) British Journal of Haematology 97, 881-888.

Kroczek (1993) Journal of Chromatography 618, 133-145.

Kronenwett et al. (1998) Blood 91, 852-862.

Leong et al. (1986) Journal of Histochemistry and Cytochemistry 34, 1645-1650.

Miyake et al. (1991) Journal of Experimental Medicine 173, 599-607.

Papayannopoulou et al., 1993, Proceedings of the National Academy of Science of the U.S.A. 90, 9374-9378.

Podolsky et al. (1993) Journal of Clinical Investigation 92,302-380.

Sambrook et al. (1989) Molecular Cloning, a laboratory manual, 2. Aufl., Cold Spring Harbor Laboratory Press, New York, S. 18.47-18.59.

Takada et al., 1989, EMBO Journal 8, 1361-1368.

Takahashi et al. (1990) In Vitro Cell Development and Biology 25, 265-274.

Wahl et al. (1994) Journal of Clinical Investigation 94, 655-662.

Yacyshyn et al., 1998, Gastroenterology 114, 1133-1142.

Yednok et al. (1992) Nature 356, 63-66.

Young et al. (1993) Proceedings of the National Academy of Science of the U.S.A. 90, 10494-10498

## Patentansprüche

1. Nukleinsäure, enthaltend eine Nukleotidsequenz, welche durch Bindung an ein Transkriptionsprodukt des für die α4-Kette des Adhäsionsmoleküls VLA-4 kodierenden Gens die Expression des Adhäsionsmoleküls VLA-4 in humanen Zellen, die aus der Gruppe, bestehend aus Monozyten, Granulozyten, Lymphomzellen, Zellen des Rhabdomyosarkoms und des malignen Melanoms ausgewählt sind, vollständig oder mindestens um 30% inhibiert.

2. Nukleinsäure nach Anspruch 1, welche Desoxyribonukleotide und/oder Ribonukleotide und/oder modifizierte Nukleotide enthält.

3. Nukleinsäure nach Anspruch 1 oder 2, die eine Antisense-Nukleinsäure ist, wobei die Nukleotidsequenz 5'-GTGGGGAACATTCAACACTA-3' ist.

4. Vektor, enthaltend mindestens eine der nach einem der Ansprüche 1 bis 3 definierten Nukleinsäuren.

5. Wirtszelle, enthaltend mindestens eine nach einem der Ansprüche 1 bis 3 definierten Nukleinsäuren oder den nach Anspruch 4 definierten Vektor.

6. Pharmazeutische Zusammensetzung, enthaltend die Nukleinsäure nach einem der Ansprüche 1 bis 3 oder den Vektor nach Anspruch 4, gegebenenfalls in Verbindung mit einem pharmazeutisch annehmbaren Lösungsmittel und/oder Träger.

7. Pharmazeutische Zusammensetzung nach Anspruch 6 zur Behandlung maligner hämatologischer Erkrankungen in Säugern.

8. Pharmazeutsiche Zusammensetzung nach Anspruch 7, wobei die maligne hämatologische Erkrankung aus der Gruppe der malignen Lymphome ausgewählt ist.

9. Pharmazeutische Zusammensetzung nach Anspruch 6 zur Hemmung der Metastasierung von Tumorzellen, die VLA-4 exprimieren, in Säugern.

10. Pharmazeutische Zusammensetzung nach Anspruch 9, wobei die Tumorzellen Zellen des Rhabdomyosarkoms und/oder des malignen Melanoms sind.

11. Pharmazeutische Zusammensetzung nach Anspruch 6 zur Behandlung pathologischer Entzündungsreaktionen in Säugern.

12. Pharmazeutische Zusammensetzung nach Anspruch 11, wobei die pathologische Entzündungsreaktion ausgewählt aus der Gruppe der akuten oder chronischen entzündlichen Erkrankungen, bestehend aus chronisch-entzündlichen Darmerkrankungen, rheumatischen Systemerkrankungen, Multipler Sklerose, Autoimmunerkrankungen, entzündliche Hauterkrankungen, "Graft-versus-host"-Reaktionen nach allogener Knochenmark- oder Blutstammzelltransplantation, Organabstoßungsreaktionen nach Transplantation, Allergien, Asthma, Virusinfektionen und Immunreaktionen bei wiederholter Applikation von Virusvektoren in der Gentherapie.

13. Pharmazeutische Zusammensetzung nach Anspruch 12, wobei die akute oder chronische entzündliche Erkrankung M. Crohn, Colitis Ulcerosa, rheumatoide Arthritis, Lupus erythematodes, Glomerulonephritis, Neurodermitis, Psoriasis, Akne oder eine Immunreaktion bei wiederholter Applikation von Adenovirusvektoren am Bronchialepithel ist.

14. Pharmazeutische Zusammensetzung nach einem der Ansprüche 7 bis 13, wobei der Säuger *Homo sapiens sapiens* ist.

## Claims

1. Nucleic acid, comprising a nucleotide sequence which inhibits expression of the adhesion molecule VLA-4 in human cells selected from the group consisting of monocytes, granulocytes, lymphoma cells, rhabdomyosarcoma cells and malignant melanoma cells completely or by at least 30% by binding to a transcription product of the gene coding for the α4 chain of the adhesion molecule VLA-4.

2. Nucleic acid according to Claim 1, which comprises deoxynucleotides and/or ribonucleotides and/or modified nucleotides.

3. Nucleic acid according to Claim 1 or 2, which is an antisense nucleic acid having the nucleotide sequence

5'-GTGGGGAACATTCAACACTA-3'.

4. Vector, comprising at least one of the nucleic acids defined according to any of Claims 1 to 3.

5. Host cell, comprising at least one of the nucleic acids defined according to any of Claims 1 to 3 or the vector defined according to Claim 4.

6. Pharmaceutical composition, comprising the nucleic acid according to any of Claims 1 to 3 or the vector according to Claim 4, where appropriate in combination with a pharmaceutically acceptable solvent and/or carrier.

7. Pharmaceutical composition according to Claim 6 for the treatment of malignant haematological disorders in mammals.

8. Pharmaceutical composition according to Claim 7, wherein the malignant haematological disorder is selected from the group of malignant lymphomas.

9. Pharmaceutical composition according to Claim 6 for inhibiting metastasizing of VLA-4-expressing tumour cells in mammals.

10. Pharmaceutical composition according to Claim 9, wherein the tumour cells are rhabdomyosarcoma cells and/or malignant melanoma cells.

11. Pharmaceutical composition according to Claim 6 for the treatment of pathological inflammatory reactions in mammals.

12. Pharmaceutical composition according to Claim 11, wherein the pathological inflammatory reactions are selected from the group of acute or chronic inflammatory disorders, consisting of chronically inflammatory bowel diseases, systemic rheumatic disorders, multiple sclerosis, autoimmune diseases, inflammatory skin diseases, graft-versus-host reactions after allogeneic transplantation of bone marrow or blood stem cells, organ rejections after transplantation, allergies, asthma, viral infections and immune responses to repeated administration of viral vectors in gene therapy.

13. Pharmaceutical composition according to Claim 12, wherein the acute or chronic inflammatory disorder is Crohn's disease, ulcerative colitis, rheumatoid arthritis, lupus erythematosus, glomerulonephritis, neurodermatitis, psoriasis, acne or an immune response to repeated administration of adenoviral vectors at the bronchial epithelium.

14. Pharmaceutical composition according to any of Claims 7 to 13, wherein the mammal is *Homo sapiens sapiens.*

## Revendications

1. Acide nucléique contenant une séquence de nucléotides qui inhibe totalement ou à 30 % au moins, par liaison à un produit de transcription du gène codant la chaîne α4 de la molécule d'adhésion VLA-4, l'expression de la molécule d'adhésion VLA-4 dans des cellules humaines qui sont choisies dans le groupe constitué de monocytes, de granulocytes, de cellules de lymphome, de cellules du rhabdomyosarcome ou du mélanome malin.

2. Acide nucléique selon la revendication 1, qui contient des désoxyribonucléotides et/ou des ribonucléotides et/ou des nucléotides modifiés.

3. Acide nucléique selon la revendication 1 ou 2, qui est un acide nucléique antisens, la séquence de nucléotides étant :

5'-GTGGGGAACATTCAACACTA-3'

4. Vecteur contenant au moins l'un des acides nucléiques définis selon l'une des revendications 1 à 3.

5. Cellule-hôte, contenant au moins l'un des acides nucléiques définis selon l'une des revendications 1 à 3 ou le vecteur défini selon la revendication 4.

6. Composition pharmaceutique, contenant l'acide nucléique selon l'une des revendications 1 à 3 ou le vecteur selon la revendication 4, le cas échéant en liaison avec un solvant et/ou un support acceptables du point de vue pharmaceutique.

7. Composition pharmaceutique suivant la revendication 6, destinée au traitement d'affections hématologiques malignes chez des mammifères.

8. Composition pharmaceutique suivant la revendication 7, l'affection hématologique maligne étant choisie dans le groupe des lymphomes malins.

9. Composition pharmaceutique suivant la revendication 6, destinée à l'inhibition de la métastase de cellules tumorales qui expriment la molécule VLA-4 chez des mammifères.

10. Composition pharmaceutique suivant la revendication 9, les cellules tumorales étant des cellules du rhabdomyosarcome et/ou du mélanome malin.

11. Composition pharmaceutique suivant la revendication 6, destinée au traitement de réactions inflammatoires pathologiques chez des mammifères.

12. Composition pharmaceutique suivant la revendication 11, la réaction inflammatoire pathologique étant choisie dans le groupe des maladies inflammatoires aiguës ou chroniques, comprenant des infections intestinales inflammatoires chroniques, des affections rhumatismales généralisées, la sclérose en plaques, des maladies auto-immunes, des maladies inflammatoires de la peau, des réactions « Graft-versus-host » après transplantation de moelle osseuse allogénique ou de cellules souches sanguines, des réactions de rejet d'organes après transplantation, des allergies, de l'asthme, des infections virales et des immunoréactions en cas d'utilisation répétée de vecteurs viraux dans la thérapie génique.

13. Composition pharmaceutique suivant la revendication 12, la maladie inflammatoire aiguë ou chronique étant la maladie de Crohn, la rectocolite hémorragique, l'arthrite rhumatoïde, le lupus érythémateux, la glomérulonéphrite, la névrodermite, le psoriasis, l'acné ou une immunoréaction en cas d'application répétée de vecteurs adénoviraux à l'épithélium bronchique.

14. Composition pharmaceutique suivant l'une des revendications 7 à 13, le mammifère étant *Homo sapiens sapiens.*
